(19) Europäisches Patentamt
European Patent Office
Office européen des brevets

(11) Numéro de publication : **0 480 800 B1**

(12) **FASCICULE DE BREVET EUROPEEN**

(45) Date de publication du fascicule du brevet : **03.08.94 Bulletin 94/31**

(51) Int. Cl.$^5$ : **C07C 37/60,** C07C 39/08

(21) Numéro de dépôt : **91402647.1**

(22) Date de dépôt : **04.10.91**

(54) **Procédé d'hydroxylation de composés phénoliques.**

(30) Priorité : **08.10.90 FR 9012345**

(43) Date de publication de la demande : **15.04.92 Bulletin 92/16**

(45) Mention de la délivrance du brevet : **03.08.94 Bulletin 94/31**

(84) Etats contractants désignés : **DE FR GB IT NL SE**

(56) Documents cités :
**FR-A- 2 266 683**
**FR-A- 2 266 684**

(73) Titulaire : **RHONE-POULENC CHIMIE**
**25, quai Paul Doumer**
**F-92408 Courbevoie Cédex (FR)**

(72) Inventeur : **Costantini, Michel**
**10, rue du Docteur Bonhomme**
**F-69003 Lyon (FR)**
Inventeur : **Dromard, Adrien**
**24, rue du Commandant Faurax**
**F-69006 Lyon (FR)**
Inventeur : **Jouffret, Michel**
**17, The Lodge**
**Kensington Park Gardens, London W11 (GB)**

(74) Mandataire : **Dutruc-Rosset, Marie-Claude et al**
**RHONE-POULENC CHIMIE**
**Service Brevets Chimie**
**25, Quai Paul Doumer**
**F-92408 Courbevoie Cédex (FR)**

EP 0 480 800 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un procédé d'hydroxylation de composés phénoliques et plus particulièrement un procédé d'hydroxylation de phénols et d'éthers de phénol, par le peroxyde d'hydrogène.

De nombreux procédés d'hydroxylation des phénols sont décrits dans l'état de la technique.

Citons, entre autres, le brevet FR-A 2 071 464 qui concerne un procédé industriel très important d'hydroxylation de phénols et d'éthers de phénols.

Ledit procédé consiste à réaliser l'hydroxylation, par l'eau oxygénée, en présence d'un acide fort. Parmi ces acides forts, l'acide sulfurique, l'acide paratoluène-sulfonique, l'acide perchlorique sont les plus utilisés.

L'hydroxylation du phénol effectuée dans les conditions décrites conduit à un mélange d'hydroquinone et de pyrocatéchine, avec prédominance de celle-ci puisque le rapport hydroquinone/pyrocatéchine varie le plus souvent entre 0,3 et 0,7.

On a proposé, selon FR-A 2 266 683, un perfectionnement à ce procédé qui consiste à effectuer l'hydroxylation, en présence d'une cétone. Il en résulte une amélioration du rendement de la réaction en hydroquinone et pyrocatéchine. Toutefois, tous les exemples décrits conduisent à une quantité plus importante de pyrocatéchine par rapport à celle d'hydroquinone.

Les procédés connus conduisent donc principalement à la pyrocatéchine.

Il s'avère que pour répondre à la demande du marché qui est fluctuante, il est important, de disposer d'un procédé industriel permettant d'accéder d'une manière prépondérante à l'hydroquinone.

Un des objectifs de l'invention est de fournir un procédé d'hydroxylation du phénol permettant d'accroître la quantité d'hydroquinone formée par rapport à la quantité de pyrocatéchine.

Un autre objectif de l'invention est de fournir dans sa variante préférée un procédé d'hydroxylation du phénol qui permette d'obtenir plus d'hydroquinone que de pyrocatéchine.

Plus particulièrement, la présente invention a pour objet un procédé d'hydroxylation de composés phénoliques de formule générale (I):

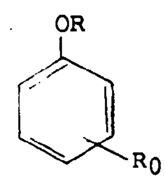

(I)

dans laquelle R et Ro, identiques ou différents représentent un atome d'hydrogène, un radical alkyle ayant de 1 à 4 atomes de carbone, un radical cyclohexyle, un radical phényle, au moyen de peroxyde d'hydrogène, en présence d'une quantité efficace d'un acide fort, ledit procédé étant caractérisé par le fait que la réaction est conduite en présence d'un composé cétonique répondant à la formule générale (II) :

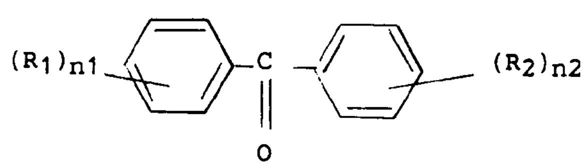

(II)

dans ladite formule (II):

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position $\alpha$ par rapport aux deux atomes de carbone portant le groupe -CO peuvent être liés ensemble par un lien valentiel ou par un groupe $-CH_2-$ formant ainsi un cycle cétonique qui peut être saturé mais aussi insaturé.

Dans l'exposé qui suit de la présente invention, on entend par "groupe électro-donneur", un groupe tel que défini par H.C. BROWN dans l'ouvrage de Jerry MARCH - Advanced Organic Chemistry, chapitre 9, pages 243 et 244.

Conformément au procédé de l'invention, on choisit un groupe électro-donneur ne réagissant pas dans les conditions d'acidité de l'invention.

A titre d'exemples de groupes électro-donneurs convenant à la présente invention, on peut citer :

- les radicaux alkyle linéaires ou ramifiés ayant de 1 à 4 atomes de carbone,

- le radical phényle,
- les radicaux alkoxy $R_3$-O- dans lesquels $R_3$ représente un radical alkyle linéaire ou ramifié ayant de 1 à 4 atomes de carbone ou le radical phényle,
- le groupe hydroxyle,
- l'atome de fluor.

On a constaté de manière inattendue, que la mise en oeuvre lors de l'hydroxylation du phénol par le peroxyde d'hydrogène, d'un composé cétonique répondant spécifiquement à la formule (II), exerce une action sur la sélectivité vis-à-vis de la formation de l'hydroquinone, en augmentant la production de ce composé au détriment de la pyrocatéchine.

Ainsi, certains composés précités permettent de faire passer le rapport molaire hydroquinone/pyrocatéchine d'une valeur inférieure à 1,0 à une valeur supérieure ou égale à 1,0. On peut citer tout particulièrement les composés cétoniques répondant à la formule générale (II) dans laquelle $R_1$ et $R_2$, identiques ou différents représentent un atome d'hydrogène ou un groupe électro-donneur, de préférence en position 4,4′ et $n_1$, $n_2$ identiques ou différents sont égaux à 0 ou 1.

On fait appel préférentiellement aux composés cétoniques répondant à la formule (II) dans laquelle $R_1$ et $R_2$ identiques ou différents représentent un atome d'hydrogène ; un radical méthyle, éthyle, tertiobutyle, phényle ; un radical méthoxy ou éthoxy ; un groupe hydroxyle, de préférence en position 3,3′ ou 4,4′.

Comme exemples spécifiques de cétones qui peuvent être utilisées dans le procédé de l'invention, on peut citer plus particulièrement :

- la benzophénone
- la méthyl-2 benzophénone
- la diméthyl-2,4 benzophénone
- la diméthyl-4,4′ benzophénone
- la diméthyl-2,2′ benzophénone
- la diméthoxy-4,4′ benzophénone
- la fluorénone
- l'hydroxy-4 benzophénone
- la dihydroxy-4-4′ benzophénone
- la benzoyl-4 biphényle

Conformément au procédé de l'invention, la présence du composé cétonique de formule (II) lors de l'hydroxylation du composé phénolique de formule (I) joue sur la régiosélectivité de la réaction.

Ledit composé est mis en oeuvre en quantité catalytique. Généralement, la quantité du composé cétonique de formule (II) exprimée en moles par mole de peroxyde d'hydrogène varie entre $1.10^{-3}$ mole et 10. Il n'est pas nécessaire de dépasser 1,0 mole de composé cétonique par mole de peroxyde d'hydrogène. Dans la pratique, la quantité de composé cétonique est le plus souvent comprise entre 0,05 et 1,0 mole par mole de peroxyde d'hydrogène.

Le peroxyde d'hydrogène mis en oeuvre selon l'invention peut être sous forme de solution aqueuse ou de solution organique.

Les solutions aqueuses étant commercialement plus facilement disponibles sont utilisées, de préférence.

La concentration de la solution aqueuse de peroxyde d'hydrogène bien que non critique en soi est choisie de façon à introduire le moins d'eau possible dans le milieu réactionnel. On utilise généralement une solution aqueuse de peroxyde d'hydrogène à au moins 20 % en poids de $H_2O_2$ et, de préférence, aux environs de 70 %.

La quantité de peroxyde d'hydrogène peut aller jusqu'à 1 mole de $H_2O_2$ pour 1 mole de composé phénolique de formule (I).

Il est cependant préférable pour obtenir un rendement industriellement acceptable d'utiliser un rapport molaire peroxyde d'hydrogène/ composé phénolique de formule (I) de 0,01 à 0,3 et, de préférence, de 0,05 à 0,10.

Afin d'avoir une vitesse de réaction suffisante, on limite la teneur initiale du milieu en eau à 20 % en poids et, de préférence, à 10 % en poids.

Les teneurs pondérales indiquées sont exprimées par rapport au mélange composé phénolique de formule (I)/peroxyde d'hydrogène/eau.

Cette eau initiale correspond à l'eau introduite avec les réactifs et notamment avec le peroxyde d'hydrogène.

Intervient dans le procédé de l'invention, un acide fort. Par acide fort, on désigne dans la présente invention, un acide présentant un pKa dans l'eau inférieur à - 0,1 et, de préférence, inférieur à - 1,0.

Le pKa est défini comme la constante de dissociation ionique du couple acide/base, lorsque l'eau est utilisée comme solvant.

Parmi les acides répondant à cette définition, il est préférable d'utiliser ceux qui sont stables vis-à-vis de

l'oxydation par le peroxyde d'hydrogène.

On peut citer plus particulièrement les oxyacides halogénés ou non tels que l'acide sulfurique, l'acide pyrosulfurique, l'acide perchlorique, l'acide nitrique, les acides halogénosulfoniques tels que l'acide fluorosulfonique, l'acide chlorosulfonique ou l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

Parmi ces acides, on utilisera de préférence l'acide perchlorique, l'acide trifluorométhanesulfonique, l'acide paratoluènesulfonique, l'acide chlorosulfonique, l'acide fluorosulfonique, l'acide méthanesulfonique.

On choisit, tout particulièrement, l'acide perchlorique ou l'acide trifluorométhanesulfonique.

La quantité d'acide exprimée par le rapport du nombre d'équivalents de protons au nombre de moles de peroxyde d'hydrogène peut varier entre environ $1.10^{-4}$ et environ 1,0.

Une variante préférée du procédé de l'invention consiste à choisir un rapport $H^+/H_2O_2$ compris entre $1.10^{-3}$ et 0,1.

Une variante préférée du procédé de l'invention consiste à ajouter un agent complexant des ions métalliques présents dans le milieu car ceux-ci sont préjudiciables au bon déroulement du procédé de l'invention, notamment dans le cas des phénols où les rendements en produits d'hydroxylation sont faibles. Par conséquent, il est préférable d'inhiber l'action des ions métalliques.

Les ions métalliques néfastes au déroulement de l'hydroxylation sont des ions de métaux de transition et plus particulièrement, les ions fer, cuivre, chrome, cobalt, manganèse et vanadium.

Les ions métalliques sont apportés par les réactifs et notamment les composés aromatiques et l'appareillage utilisé. Pour inhiber l'action de ces ions métalliques, il suffit de conduire la réaction en présence d'un ou plusieurs agents complexants stables vis-à-vis du peroxyde d'hydrogène et donnant des complexes ne pouvant être décomposés par les acides forts présents et dans lesquels le métal ne peut plus exercer d'activité chimique.

A titre d'exemples non limitatifs d'agents complexants, on peut faire appel, notamment, aux divers acides phosphoriques tels que, par exemple, l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

On peut également mettre en oeuvre les esters des acides précités et l'on peut mentionner, plus particulièrement, les orthophosphates de mono- ou dialkyle, de mono-ou dicycloalkyle, de mono- ou dialkylaryle, par exemple, le phosphate d'éthyle ou de diéthyle, le phosphate d'hexyle, le phosphate de cyclohexyle, le phosphate de benzyle.

La quantité d'agent complexant dépend de la teneur du milieu réactionnel en ions métalliques.

La quantité d'agent complexant exprimée en nombres de moles d'agent complexant par mole de peroxyde d'hydrogène varie avantageusement entre 0,0001 et 0,01.

Une autre variante d'exécution du procédé de l'invention consiste à conduire le procédé d'hydroxylation de composés phénoliques de formule générale (I), au moyen du peroxyde d'hydrogène, en présence d'une quantité efficace d'un sel de métal alcalin ou alcalino-terreux d'un acide fort et d'une quantité efficace d'au moins un oxacide du phosphore, ledit procédé étant caractérisé par le fait que la réaction est conduite en présence du composé cétonique répondant à la formule générale (II).

Par sel d'acide fort, on entend un acide présentant un pka dans l'eau inférieur à - 0,1 et, de préférence, inférieur à - 1,0.

Parmi les sels des acides répondant à cette définition, il est préférable d'utiliser les sels de métaux alcalins ou alcalino-terreux des acides stables vis-à-vis de l'oxydation par le peroxyde d'hydrogène.

Ainsi, les sels de métaux alcalins ou alcalino-terreux des acides forts précités conviennent tout-à-fait bien.

Par sels de métaux alcalins, on entend dans le présent texte les sels neutres des acides définis précédemment de lithium, de sodium, de potassium, de rubidium et de césium.

On préfère le plus souvent utiliser les sels de sodium ou de potassium et encore plus préférentiellement, pour des raisons économiques, les sels de sodium.

Parmi ces différents sels, on peut citer, parmi les préférés, le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

Par sels de métaux alcalino-terreux, on entend dans le présent texte les sels neutres des acides définis précédemment de béryllium, de magnésium, de calcium, de strontium et de baryum.

On préfère le plus souvent utiliser les sels de magnésium, de calcium et de baryum.

Parmi ces différents sels de métaux alcalino-terreux, on mettra en oeuvre, préférentiellement, le sulfate de calcium, le sulfate de magnésium, le perchlorate de calcium, le perchlorate de magnésium, le trifluorométhanesulfonate de calcium, le trifluorométhanesulfonate de magnésium, le paratoluènesulfonate de calcium,

le paratoluènesulfonate de magnésium, le fluorosulfonate de calcium, le fluorosulfonate de magnésium, le méthanesulfonate de calcium, le méthanesulfonate de magnésium.

On peut utiliser des mélanges de plusieurs sels de métaux alcalins ou alcalino-terreux

On peut également préparer in-situ les sels de métaux alcalins ou alcalino-terreux par exemple en chargeant des quantités stoechiométriques d'acide et d'oxyde ou hydroxyde de ces métaux.

Les oxacides du phosphore sont plus particulièrement les composés à fonction acide du phosphore au degré d'oxydation 5.

On peut également utiliser des composés à fonction acide du phosphore au degré d'oxydation 3, qui seront oxydés dans le milieu par le peroxyde d'hydrogène en composés correspondants du phosphore V ; mais cela ne présente pas d'intérêt particulier, tout en ayant l'inconvénient de consommer une partie du peroxyde d'hydrogène.

Parmi ces oxacides du phosphore V, on peut citer, par exemple, l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

Les plus couramment utilisés sont, pour des questions pratiques et économiques, l'acide orthophosphorique, l'acide pyrophosphorique et l'acide (hydroxy-1 éthylidène)diphosphonique.

La quantité de sel de métal alcalin ou alcalino-terreux utilisée dans le procédé de l'invention peut varier dans de larges limites.

Généralement, cette quantité est exprimée en rapport molaire sel de métal alcalin ou alcalino-terreux/peroxyde d'hydrogène. Ce rapport est le plus souvent compris entre 0,001 et 0,10 et, de préférence, entre 0,005 et 0,05.

La quantité d'oxacide du phosphore exprimée en rapport molaire oxacide du phosphore/peroxyde d'hydrogène est le plus souvent comprise entre 0,001 et 0,20 et de préférence entre 0,05 et 0,10.

En ce qui concerne les conditions de mise en oeuvre du peroxyde d'hydrogène et du composé cétonique répondant à la formule (II), celles-ci correspondent à ce qui est décrit précédemment.

Conformément au procédé de l'invention, on réalise l'hydroxylation du composé phénolique de formule (I) à une température qui peut être comprise entre 45°C et 150°C.

Une variante préférée du procédé de l'invention consiste à choisir la température entre 45°C et 75°C.

La réaction est conduite avantageusement sous pression atmosphérique.

Le procédé d'hydroxylation est généralement mis en oeuvre sans solvant autre que celui qui provient des réactifs, comme le solvant du peroxyde d'hydrogène.

La réaction peut cependant également être réalisée dans un solvant du composé phénolique (I).

Les solvants utilisés doivent êtres stables en présence de peroxyde d'hydrogène.

On peut citer des solvants non polaires comme les hydrocarbures alipathiques chlorés, par exemple le dichlorométhane, le tétrachlorométhane, le dichloroéthane.

On peut citer des solvants peu polaires comme les alcools et les éthers, par exemple le méthanol, le tertiobutanol, l'isopropanol, l'éthanol, le méthyl-tertiobutyléther ou très polaires comme l'eau.

D'un point de vue pratique, le procédé selon l'invention est simple à mettre en oeuvre de façon continue ou discontinue.

D'un manière préférée, on choisit l'ordre des réactifs suivants: on introduit le composé phénolique de formule (I), éventuellement l'agent complexant, l'acide fort puis le composé cétonique de formule (II).

On porte le milieu réactionnel à la température désirée puis, l'on ajoute la solution de peroxyde d'hydrogène, de manière progressive.

En fin de réaction, le composé phénolique non transformé, et le cas échéant le composé cétonique de formule (II), sont séparés des produits d'hydroxylation par les moyens usuels, notamment, par distillation et sont renvoyés dans la zone réactionnelle.

Parmi les composés phénoliques de formule (I) qui pourront être mis en oeuvre dans le procédé de l'invention, on peut citer à titre non limitatif, le phénol, l'anisole, l'orthocrésol, le paracrésol, le métacrésol, le tertiobutyl-4 phénol, le méthoxy-2 phénol, le méthoxy-4 phénol.

Le présent procédé convient tout particulièrement bien à la préparation d'hydroquinone et de pyrocatéchol à partir du phénol.

Les exemples qui suivent, illustrent l'invention sans toutefois la limiter.

Dans les exemples, les abréviations suivantes signifient :

$$TT = \frac{\text{nombre de moles de peroxyde d'hydrogène transformées}}{\text{nombre de mole de peroxyde d'hydrogène introduites}} \%$$

$$RT_{HQ} = \frac{\text{nombre de moles d'hydroquinone formées}}{\text{nombre de moles de peroxyde d'hydrogène transformées}} \%$$

$$RT_{PC} = \frac{\text{nombre de moles de pyrocatéchine formées}}{\text{nombre de moles de peroxyde d'hydrogène transformées}} \%$$

## EXEMPLE 1

### Essai comparatif a

Dans un ballon en verre de 100 ml muni d'une agitation centrale, d'un réfrigérant, d'une ampoule de coulée et d'un thermomètre, on charge :

- 79 g de phénol,
- 0,0816 g d'acide trifluorométhanesulfonique,
- 0,058 g d'acide pyrophosphorique,
- 3,14 g de benzophénone.

On porte le mélange réactionnel à 75°C, tout en le maintenant sous agitation à 1200 tours/mn.

On introduit par l'intermédiaire de l'ampoule de coulée, en 2 minutes, 2,064 g d'une solution aqueuse de peroxyde d'hydrogène à 70,5 % en poids.

On maintient le mélange réactionnel sous agitation, à 75°C.

Le taux de transformation est de 100 %, après 30 minutes.

On refroidit alors le mélange réactionnel et l'on effectue le dosage des produits de réaction : le peroxyde d'hydrogène résiduel est dosé par iodométrie et les diphénols formés sont dosés par chromatographie liquide, haute performance.

Les résultats obtenus sont les suivants:

$RT_{HYDROQUINONE}$= 42,4 %
$RT_{PYROCATECHINE}$ = 41,8 %%
Rapport HQ/PC = 1,01

A titre comparatif, on reproduit l'exemple 1 mais en l'absence de benzophénone.

La réaction est terminée au bout de 1 heure 50 minutes.

Les rendements en hydroquinone et en pyrocatéchine s'élèvent respectivement à 34 % et 49,6 % par rapport au peroxyde d'hydrogène transformé. Le rapport entre l'hydroquinone formée et la pyrocatéchine est de 0,685.

Il ressort de la comparaison de l'exemple 1 et de l'essai a que la présence de la benzophénone joue sur la régiosélectivité de la réaction et permet donc d'obtenir davantage d'hydroquinone.

## EXEMPLE 2

### Essai comparatif b :

Dans un ballon tel que décrit à l'exemple 1, on charge :

- 30,40 g (0,323 mol) de phénol,
- 0,0308 g d'une solution aqueuse d'acide perchlorique à 70 % en poids,
- 2,18 g de benzophénone.
- 0,0161 g d'acide pyrophosphorique

On porte le mélange réactionnel à 75°C tout en le maintenant sous agitation à 1200 tours/mn.

On introduit par l'intermédiaire de l'ampoule de coulée, en 2 minutes, 0,806 g d'une solution aqueuse de peroxyde d'hydrogène à 70,5 % en poids.

On maintient le mélange réactionnel sous agitation, à 75°C.

Le taux de transformation est de 100 %, après 25 minutes.

On refroidit le mélange réactionnel et l'on effectue le dosage des produits de la réaction comme dans l'exemple 1.

Les résultats obtenus sont les suivants :

$RT_{HYDROQUINONE}$ = 41,6 %
$RT_{PYROCATECHINE}$ = 43,2 %
Rapport HQ/PC = 0,96

A titre comparatif, on reproduit l'exemple 2 mais en l'absence de benzophénone.

La réaction est terminée au bout de 1 heure.

Les rendements en hydroquinone et en pyrocatéchine s'élèvent respectivement à 35,2 % et 49,6 % par rapport au peroxyde d'hydrogène transformé. Le rapport entre l'hydroquinone formé et la pyrocatéchine est

de 0,71.

EXEMPLES 3 A 7

Essais c et d

On conduit une série d'essais selon le mode opératoire de l'exemple 2 mais en mettant en oeuvre d'autres composés cétoniques de formule (II) tels que :

- l'hydroxy-4 benzophénone (exemple 3),
- la dihydroxy-4,4′ benzophénone (exemple 4),
- la diphényl-4,4′ benzophénone (exemple 5),
- la fluorénone-9 (exemple 6),
- le benzoyl-4 biphényle (exemple 7).

La température de la réaction est de 75°C.

Les quantités de réactifs mis en jeu et les résultats obtenus sont consignés dans le tableau (I) suivant.

On donne également dans le tableau I, les résultats obtenus pour la mise en oeuvre d'une benzophénone porteuse d'un groupe électroattracteur nitro ou cyano.

Tableau I

**HYDROXYLATION DU PHENOL PAR $H_2O_2/HClO_4/H_4P_2O_7$/COMPOSE CETONIQUE DE FORMULE (II)**

| Réf. | Composé cétonique (II) | Rapport molaire $H_2O_2$/PHENOL | Rapport molaire $HClO_4/H_2O_2$ | Rapport molaire $H_4P_2O_7/H_2O_2$ | Rapport molaire composé cétonique (II)/$H_2O_2$ | Durée | TT | $RT_{HQ}$ | $RT_{PC}$ | Rapport HQ/PC |
|---|---|---|---|---|---|---|---|---|---|---|
| 3 | hydroxy-4 benzophénone | $5,2.10^{-2}$ | $1,31.10^{-2}$ | $6.10^{-3}$ | 0,670 | 20 mn | 100 | 43,0 | 38,0 | 1,13 |
| 4 | dihydroxy-4,4' benzophénone | $5,1.10^{-2}$ | $1,24.10^{-2}$ | $7.10^{-3}$ | 0,196 | 110 mn | 100 | 39,2 | 39,9 | 0,98 |
| 5 | diphényl-4,4' benzophénone | $5,1.10^{-2}$ | $1,39.10^{-2}$ | $6.10^{-3}$ | 0,182 | 70 mn | 99,9 | 41,3 | 44,4 | 0,93 |
| 6 | fluorénone-9 | $5,0.10^{-2}$ | $1,42.10^{-2}$ | $7.10^{-3}$ | 0,199 | 50 mn | 100 | 42,5 | 43,7 | 0,97 |
| 7 | benzoyl-4 biphényle | $5,2.10^{-2}$ | $1,25.10^{-2}$ | $7.10^{-3}$ | 0,192 | 75 mn | 100 | 41,2 | 42,2 | 0,98 |
| c | nitro-4 benzophénone | $5,3.10^{-2}$ | $1,36.10^{-2}$ | $6.10^{-3}$ | 0,190 | 105 mn | 100 | 36,4 | 47,7 | 0,77 |
| d | cyano-4 benzophénone | $4,9.10^{-2}$ | $1,60.10^{-2}$ | $10^{-2}$ | 0,580 | 55 mn | 100 | 35,5 | 46,7 | 0,76 |

Il ressort de l'examen du tableau I que la nature du groupe influe d'une manière surprenante sur la régiosélectivité de la réaction et que seules les benzophénones porteuses d'un groupe électro-donneur permettent d'obtenir un mélange d'hydroquinone et de pyrocatéchine contenant une quantité prépondérante d'hydroquinone.

EXEMPLE 8

Essai e

Dans un ballon à 3 cols de 250 ml, muni d'un thermomètre, d'un dispositif d'agitation, d'un réfrigérant ascendant, d'un système de chauffage, et d'une arrivée d'azote, on charge après purge à l'azote :

- 94 g de phénol fondu,
- 0,92 g d'acide phosphorique à 85 % en poids,
- 1,82 g de benzophénone,
- 1,03 g d'une solution aqueuse d'acide perchlorique à 70 % en poids.

On maintient le contenu du ballon à 45°C puis l'on ajoute 2,02 g d'une solution aqueuse de peroxyde d'hydrogène à 84,7 % en poids.

Le taux de transformation est de 100 %, après 50 minutes

La masse réactionnelle est neutralisée par une solution N/2 de potasse dans le méthanol, puis on la dilue par addition d'un volume de méthanol.

Les produits de la réaction et la benzophénone sont ensuite dosés par chromatographie gazeuse. Les résultats suivants ont été obtenus :

- benzophénone : 1,82 g
- hydroquinone : 2,39 g
- pyrocatéchine : 2,33 g

Les rendements en hydroquinone et pyrocatéchine s'élèvent respectivement à 42,8 et 41,7 par rapport au peroxyde d'hydrogène mis en oeuvre. Le rapport entre l'hydroquinone formée et la pyrocatéchine est de 1,02.

A titre comparatif, on répète l'exemple 8 mais en l'absence de benzophénone.

Le taux de transformation est de 100 % au bout de 3 heures 12 minutes.

Les rendements en hydroquinone et en pyrocatéchine par rapport au peroxyde d'hydrogène mis en oeuvre s'élèvent respectivement de 33,7 % et de 52,3 %. Le rapport hydroquinone/pyrocatéchine est de 0,64.

La comparaison des résultats de l'exemple 8 et de l'essai e permet de constater l'influence de la benzophénone sur la répartition des produits formés.

EXEMPLES 9 ET 10

Essai f

Dans cette série d'exemples, on fait varier les quantités de benzophénone engagée selon les indications du tableau (II).

On opère selon le mode opératoire de l'exemple 8, les conditions opératoires étant les suivantes :

- 1 mole de phénol fondu
- 0,00049 mole d'acide phosphorique à 85 %,
- 0,050 mole de peroxyde d'hydrogène à 84,7 % en poids,
- 0,000625 mole d'acide perchlorique à 70 % en poids.

Les rapports molaires des réactifs sont les suivants :

- rapport molaire $H_2O_2$/phénol = $5.10^{-2}$,
- rapport molaire $HClO_4/H_2O_2$ = $1,25.10^{-2}$,
- rapport molaire $H_3PO_4/H_2O_2$ = $9,8.10^{-3}$

L'essai f correspond à l'essai comparatif avec absence de benzophénone.

Les résultats obtenus sont consignés dans le tableau (II).

Tableau II

Hydroxylation du phénol par $H_2O_2/HClO_4/H_3PO_4$/benzophénone

| Réf. | Rapport molaire benzophénone/$H_2O_2$ | Durée mn | TT | $RT_{HQ}$ | $RT_{PC}$ | Rapport HQ/PC |
|---|---|---|---|---|---|---|
| 9 | 0,20 | 30 | 100 | 43,9 | 42,3 | 1,03 |
| 10 | 0,33 | 30 | 100 | 45,0 | 42,0 | 1,06 |
| f | - | 90 | 100 | 35,0 | 50,0 | 0,70 |

EXEMPLE 11

On opère comme dans l'exemple 8, avec les réactifs et les conditions opératoires des exemples 9 et 10, mais avec les rapports molaires benzophénone/$H_2O_2$ de 0,5 et $H_2O_2$/phénol de 0,1.

Le taux de transformation est de 100 % après 30 mn.

Les résultats obtenus sont les suivants :

$RT_{HYDROQUINONE}$ = 37,5 %
$RT_{PYROCATECHINE}$ = 34,8 %
Rapport HQ/PC = 1,07

EXEMPLE 12

On opère comme dans la série d'exemples 9 et 10, mais en remplaçant la benzophénone par la diméthoxy-4,4′ benzophénone, avec un rapport molaire $H_2O_2$/phénol de 0,1.

Le taux de transformation est de 100 % après 1 heure.

Les résultats obtenus sont les suivants :

$RT_{HYDROQUINONE}$ = 40,0
$RP_{PYROCATECHINE}$ = 43,6
Rapport HQ/PC = 0,92

EXEMPLE 13

On opère comme dans la série d'exemples 9 et 10, mais en remplaçant la benzophénone par la diméthyl-4,4′ benzophénone avec un rapport molaire $H_2O_2$/phénol de 0,2.

Le taux de transformation est de 100 %, après 45 minutes.

Les résultats obtenus sont les suivants :

$RT_{HYDROQUINONE}$ = 47,2
$RT_{PYROCATECHINE}$= 41,7
Rapport HQ/PC = 1,13

EXEMPLE 14

Essai comparatif q

Dans un ballon en verre de 100 ml muni d'une agitation centrale, d'un réfrigérant, d'une ampoule de coulée et d'un thermomètre, on charge :

- 41,12 g de phénol,

- 0,081 g de perchlorate de soude $NaClO_4$, $H_2O$
- 0,236 g d'acide pyrophosphorique,
- 0,798 g de benzophénone.

On porte le mélange réactionnel à 75°C, tout en le maintenant sous agitation à 1200 tours/mn.

On introduit par l'intermédiaire de l'ampoule de coulée, en 2 minutes, 1,083 g d'une solution aqueuse de peroxyde d'hydrogène à 70,25 % en poids.

On maintient le mélange réactionnel sous agitation, à 75°C.

Le taux de transformation est de 96,1 %, après 320 minutes.

On refroidit alors le mélange réactionnel et l'on effectue le dosage des produits de réaction : le peroxyde d'hydrogène résiduel est dosé par iodométrie et les diphénols formés sont dosés par chromatographie liiquide, haute performance.

Les résultats obtenus sont les suivants :

| | |
|---|---|
| $RT_{HYDROQUINONE}$ = | 38,0 % |
| $RT_{PYROCATECHINE}$ = | 41,0 % |
| Rapport HQ/PC = | 0,93 |

A titre comparatif, on reproduit l'exemple 14 mais en l'absence de benzophénone.

Dans le ballon tel que précédemment décrit à l'exemple 14, on charge :

- 41,65 g de phénol,
- 0,078 g de perchlorate de soude $NaClO_4$, $H_2O$
- 0,221 g d'acide pyrophosphorique,

On porte le mélange réactionnel à 75°C tout en le maintenant sous agitation à 1200 tours/mn.

On introduit par l'intermédiaire de l'ampoule de coulée, en 2 minutes, 1,141 g d'une solution aqueuse de peroxyde d'hydrogène à 70,25 % en poids.

On maintient le mélange réactionnel sous agitation, à 75°C.

Le taux de transformation est de 98,6 %, après 260 minutes.

On refroidit le mélange réactionnel et l'on effectue le dosage des produits de la réaction comme dans l'exemple 14.

Les résultats obtenus sont les suivants :

| | |
|---|---|
| $RT_{HYDROQUINONE}$ = | 33,5 % |
| $RT_{PYROCATECHINE}$ = | 50,5 % |
| Rapport HQ/PC = | 0,66 |

Lorsque l'on compare les résultats obtenus dans l'exemple 14 et l'essai comparatif g, on note une forte influence de la présence de la benzophénone sur la régiosélectivité de la réaction.

## EXEMPLES 15 ET 16

Dans cette série d'exemples, on met en évidence l'influence de la température sur la sélectivité de la réaction d'hydroxylation du phénol en hydroquinone.

La cétone utilisée est la benzophénone.

On opère selon le mode opératoire de l'exemple 1, à la différence près que l'on utilise l'acide perchlorique à la place de l'acide trifluorométhanesulfonique.

Les quantités de réactifs et les conditions opératoires sont consignées dans le tableau (III) suivant.

On donne également dans le tableau (III), les résultats obtenus.

Tableau III

**HYDROXYLATION DU PHENOL PAR $H_2O_2/HClO_4/H_4P_2O_7$/benzophénone**

| Réf. | Température °C | Rapport molaire $H_2O_2$/PHENOL | Rapport molaire $HClO_4/H_2O_2$ | Rapport molaire $H_4P_2O_7/H_2O_2$ | Rapport molaire benzophénone/$H_2O_2$ | Durée | TT | $RT_{HQ}$ | $RT_{PC}$ | Rapport HQ/PC |
|---|---|---|---|---|---|---|---|---|---|---|
| 15 | 45 | $5,1.10^{-2}$ | $1,20.10^{-2}$ | $5.10^{-3}$ | 0,20 | 120 mn | 100 | 42,5 | 41,5 | 1,02 |
| 16 | 75 | $5,2.10^{-2}$ | $1,25.10^{-2}$ | $5.10^{-3}$ | 0,19 | 45 mn | 100 | 40,1 | 43,3 | 0,93 |

EXEMPLES 17 ET 18

Dans cette série d'exemples, on met en évidence l'influence de la concentration en acide sur la sélectivité de la réaction d'hydroxylation du phénol en hydroquinone.

La cétone utilisée est la benzophénone.

On opère selon le mode opératoire de l'exemple 1, à la différence près que l'on utilise l'acide perchlorique à la place de l'acide trifluorométhanesulfonique.

Les quantités de réactifs et les conditions opératoires sont consignés dans le tableau (IV) suivant.

On donne également dans le tableau (IV), les résultats obtenus.

Tableau IV

**HYDROXYLATION DU PHENOL PAR $H_2O_2/HClO_4/H_4P_2O_7$/benzophénone**

| Réf. | Température °C | Rapport molaire $H_2O_2$/PHENOL | Rapport molaire $HClO_4/H_2O_2$ | Rapport molaire $H_4P_2O_7/H_2O_2$ | Rapport molaire benzophénone/$H_2O_2$ | Durée | TT | $RT_{HQ}$ | $RT_{PC}$ | Rapport HQ/PC |
|---|---|---|---|---|---|---|---|---|---|---|
| 17 | 45 | $4,9.10^{-2}$ | $0,4.10^{-2}$ | $6,0.10^{-3}$ | 0,206 | 5 h 45 | 95 | 42,5 | 41,5 | 1,02 |
| 18 | 45 | $5,1.10^{-2}$ | $1,6.10^{-2}$ | $6,0.10^{-3}$ | 0,197 | 2 h | 100 | 43,5 | 48,0 | 0,91 |

EXEMPLE 19

Dans cet exemple, on met en évidence que la présence d'un agent complexant peut être superfétatoire lorsque l'on fait appel à des réactifs d'une grande pureté.

On met en oeuvre du phénol commercialisé par la Société CARLO ERBA ayant une pureté supérieure à 99, 5 %.

On opère selon le mode opératoire de l'exemple 1 mais avec les quantités de réactifs suivantes :

- 0,5 mole de phénol
- 0,025 mole de benzophénone
- 0,0244 mole de peroxyde d'hydrogène à 71,2 % en poids,
- $3,3.10^{-4}$ mole d'acide perchlorique à 70 % en poids.

Les rapports molaires des réactifs sont les suivants :

- rapport molaire $H_2O_2$/phénol = $4,9.10^{-2}$,
- rapport molaire $HClO_4/H_2O_2$ = $1,36.10^{-2}$,
- rapport molaire benzophénone/$H_2O_2$ = 1,03.

Les résultats obtenus sont consignés dans le tableau (V).

Tableau V

Hydroxylation du phénol par $H_2O_2/HClO_4$/benzophénone

| Réf. | Température °C | Durée mn | TT | $RT_{HQ}$ | $RT_{PC}$ | Rapport HQ/PC |
|---|---|---|---|---|---|---|
| 19 | 75 | 30 | 100 | 42,5 | 42,0 | 1,01 |

**Revendications**

**1.** Procédé d'hydroxylation de composés phénoliques de formule générale (I) :

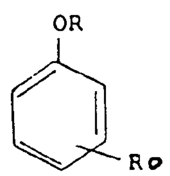

(I)

dans laquelle R et Ro, identiques ou différents représentent un atome d'hydrogène, un radical alkyle ayant de 1 à 4 atomes de carbone, un radical cyclohexyle, un radical phényle, au moyen de peroxyde d'hydrogène, en présence d'une quantité efficace d'un acide fort, ledit procédé étant caractérisé par le fait que la réaction est conduite en présence d'un composé cétonique répondant à la formule générale (II) :

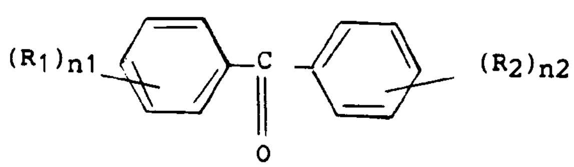

(II)

dans ladite formule (II) :

- $R_1$ et $R_2$ identiques ou différents représentent un atome d'hydrogène ou un groupe électro-donneur,
- $n_1$, $n_2$ identiques ou différents est un nombre égal à 0, 1, 2 ou 3,
- éventuellement les deux atomes de carbone situés en position $\alpha$ par rapport aux deux atomes de

carbone portant le groupe -CO peuvent être liés ensemble par un lien valentiel ou par un groupe -$CH_2$- formant ainsi un cycle cétonique qui peut être saturé mais aussi insaturé.

2. Procédé selon la revendication 1 caractérisé par le fait que le composé cétonique répond à la formule générale (II) dans laquelle $R_1$, $R_2$ identiques ou différents représentent :
   - des radicaux alkyle linéaires ou ramifiés ayant de 1 à 4 atomes de carbone,
   - un radical phényle,
   - des radicaux alkoxy $R_3$-O- dans lesquels $R_3$ représente un radical alkyle linéaire ou ramifié ayant de 1 à 4 atomes de carbone ou le radical phényle,
   - un groupe hydroxyle,
   - un atome de fluor.

3. Procédé selon l'une des revendications 1 et 2 caractérisé par le fait que le composé cétonique répond à la formule générale (II) dans laquelle $R_1$, $R_2$ identiques ou différents représentent un atome d'hydrogène ou un groupe électro-donneur en position 4,4′ et $n_1$, $n_2$ identiques ou différents sont égaux à 0 ou 1.

4. Procédé selon l'une des revendications 1 à 3 caractérisé par le fait que le composé cétonique répond à la formule générale (II) dans laquelle $R_1$, $R_2$ identiques ou différents représentent un atome d'hydrogène ; un radical méthyle, éthyle, tertiobutyle, phényle ; un radical méthoxy ou éthoxy ; un groupe hydroxyle, de préférence en position 3,3′ ou 4,4′.

5. Procédé selon l'une des revendications 1 à 4 caractérisé par le fait que le composé cétonique de formule générale (II) est :
   - la benzophénone
   - la méthyl-2 benzophénone
   - la diméthyl-2,4 benzophénone
   - la diméthyl-4,4′ benzophénone
   - la diméthyl-2,2′ benzophénone
   - la diméthoxy-4,4′ benzophénone
   - la fluorénone
   - l'hydroxy-4 benzophénone
   - la dihydroxy-4-4′ benzophénone
   - la benzoyl-4 biphényle

6. Procédé selon l'une des revendications 1 à 5 caractérisé par le fait que la quantité de composé cétonique de formule (II) est d'au moins $1.10^{-3}$ mole par mole de peroxyde d'hydrogène, de préférence, comprise entre $1.10^{-3}$ mole et 10 moles par mole de peroxyde d'hydrogène et encore plus préférentiellement entre 0,05 et 1,0 mole.

7. Procédé selon l'une des revendications 1 à 6 caractérisé par le fait que l'acide fort est un acide présentant un pka dans l'eau inférieur à - 0,1, de préférence inférieur à - 1,0.

8. Procédé selon l'une des revendications 1 à 7 caractérisé par le fait que l'acide fort est un oxyacide halogéné ou non tel que l'acide sulfurique, l'acide pyrosulfurique, l'acide perchlorique, l'acide nitrique ou un acide sulfonique tel que l'acide fluorosulfonique, l'acide chlorosulfonique, l'acide trifluorométhanesulfonique, l'acide méthanesulfonique, l'acide éthanesulfonique, l'acide éthanedisulfonique, l'acide benzènesulfonigue, les acides benzènedisulfoniques, les acides toluènesulfoniques, les acides naphtalènesulfoniques et les acides naphtalènedisulfoniques.

9. Procédé selon l'une des revendications 1 à 8 caractérisé par le fait que l'acide fort est l'acide perchlorique ou l'acide trifluorométhanesulfonique.

10. Procédé selon l'une des revendications 1 à 9 caractérisé par le fait que la quantité d'acide fort est telle que le rapport $H^+/H_2O_2$ est compris entre $1.10^{-4}$ et 1,0, de préférence, entre $1.10^{-3}$ et 0,1.

11. Procédé selon l'une des revendications 1 à 6 caractérisé par le fait que l'on opère en présence d'une quantité efficace d'un sel de métal alcalin ou alcalino-terreux d'un acide fort et d'une quantité efficace d'au moins un oxacide du phosphore.

12. Procédé selon la revendication 11 caractérisé par le fait que le sel de métal alcalin ou alcalino-terreux d'un acide fort est un sel d'un oxyacide halogéné ou non tel que l'acide sulfurique, l'acide pyrosulfurique, l'acide perchlorique, l'acide nitrique ou un acide sulfonique tel que l'acide fluorosulfonique, l'acide chlorosulfonique, l'acide trifluorométhanesulfonique, l'acide méthanesulfonique, l'acide éthanesulfonique, l'acide éthanedisulfonique, l'acide benzènesulfonique, les acides benzènedisulfoniques, les acides toluènesulfoniques, les acides naphtalènesulfoniques et les acides naphtalènedisulfoniques.

13. Procédé selon l'une des revendications 11 et 12 caractérisé par le fait que le sel de métal alcalin est le sulfate disodique, le perchlorate de sodium, le trifluorométhanesulfonate de sodium, le paratoluènesulfonate de sodium, le chlorosulfonate de sodium, le fluorosulfonate de sodium, le méthanesulfonate de sodium.

14. Procédé selon l'une des revendications 11 à 13 caractérisé par le fait que le sel de métal alcalino-terreux est le sulfate de calcium le sulfate de magnésium, le perchlorate de calcium, le perchlorate de magnésium, le trifluorométhanesulfonate de calcium, le trifluorométhanesulfonate de magnésium, le paratoluènesulfonate de calcium, le paratoluènesulfonate de magnésium, le fluorosulfonate de calcium, le fluorosulfonate de magnésium, le méthanesulfonate de calcium, le méthanesulfonate de magnésium.

15. Procédé selon la revendication 11 caractérisé par le fait que les oxacides du phosphore sont les composés à fonction acide du phosphore au degré d'oxydation 5.

16. Procédé selon la revendication 15, caractérisé par le fait que les oxacides du phosphore V sont l'acide orthophosphorique, l'acide métaphosphorique, l'acide pyrophosphorique, les acides polyphosphoriques, les acides phosphoniques tels que l'acide (hydroxy-1 éthylidène)diphosphonique, l'acide phosphonique, l'acide éthylphosphonique, l'acide phénylphosphonique.

17. Procédé selon l'une des revendications 15 ou 16, caractérisé par le fait que les oxacides du phosphore sont l'acide orthophosphorique, l'acide pyrophosphorique et l'acide (hydroxy-1 éthylidène)-diphosphonique.

18. Procédé selon l'une des revendications 11 à 17, caractérisé par le fait que la quantité de sel de métal alcalin ou alcalino-terreux, exprimée en rapport molaire sel de métal alcalin ou alcalino-terreux/-peroxyde d'hydrogène est compris entre 0,001 et 0,10, de préférence, entre 0,005 et 0,05.

19. Procédé selon l'une des revendications 11 à 18, caractérisé par le fait que la quantité d'oxacide du phosphore exprimée en rapport molaire oxacide du phosphore/peroxyde d'hydrogène est comprise entre 0,001 et 0,20, de préférence, entre 0,005 et 0,10.

20. Procédé selon l'une des revendications 1 à 19, caractérisé par le fait que le rapport molaire $H_2O_2$/composé phénolique de formule (I) est compris entre 0,01 et 0,3, de préférence, entre 0,05 et 0,10.

21. Procédé selon l'une des revendications 1 à 11 caractérisé par le fait que l'on opère en présence d'un agent complexant des ions de métaux de transition, stables dans les conditions de réaction tels que les acides phosphoriques, les acides polyphosphoriques, les acides phosphoniques et leurs esters-acides.

22. Procédé selon l'une des revendications 1 à 21 caractérisé par le fait que l'on opère à une température comprise entre 45°C et 150°C, de préférence, entre 45°C et 75°C.

23. Procédé selon l'une des revendications 1 à 22, caractérisé par le fait que le composé phénolique de formule (I) est le phénol, l'anisole, l'orthocrésol, le paracrésol, le métacrésol, le tertiobutyl-4 phénol, le méthoxy-2 phénol, le méthoxy-4 phénol.

24. Procédé selon l'une des revendications 1 à 23 caractérisé par le fait que le composé phénolique de formule (I) est le phénol.

## Patentansprüche

1. Verfahren zur Hydroxylierung von Phenolverbindungen der allgemeinen Formel (I):

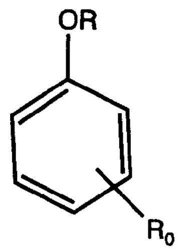

(I)

in der R und $R_o$, identisch oder verschieden, ein Wasserstoffatom, einen Alkylrest mit 1 bis 4 Kohlenstoffatomen, einen Cyclohexylrest oder einen Phenylrest darstellen, mit Hilfe von Wasserstoffperoxid in Gegenwart einer wirksamen Menge einer starken Säure, dadurch gekennzeichnet, daß die Reaktion in Gegenwart einer Keto-Verbindung der allgemeinen Formel (II)

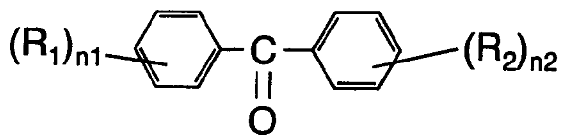

(II)

durchgeführt wird, in der:

- $R_1$ und $R_2$, identisch oder verschieden, ein Wasserstoffatom oder eine Elektronendonatorverbindung darstellen,
- $n_1$ und $n_2$, identisch oder verschieden, eine Zahl gleich 0, 1, 2 oder 3 sind,
- die beiden Kohlenstoffatome in α-Position zu den beiden Kohlenstoffatomen an der CO-Gruppe gegebenenfalls durch eine Valenzbindung oder eine $-CH_2$-Gruppe miteinander verbunden sein können und so einen Cyclus mit Keto-Funktion bilden, der gesättigt oder auch ungesättigt sein kann.

2. Verfahren nach Anspruch 1, dadurch gekennzeichnet, daß die Keto- Verbindung der allgemeinen Formel (II) entspricht, in der $R_1$ und $R_2$, identisch oder verschieden,
   - lineare oder verzweigte Alkylreste mit 1 bis 4 Kohlenstoffatomen,
   - einen Phenylrest,
   - $R_3$-O-Alkoxyreste, wobei $R_3$ einen linearen oder verzweigten Alkylrest mit 1 bis 4 Kohlenstoffatomen oder ein Phenylrest ist,
   - eine Hydroxylgruppe
   - ein Fluoratom

   darstellen.

3. Verfahren nach einem der Ansprüche 1 und 2, dadurch gekennzeichnet, daß die Keto-Verbindung der allgemeinen Formel (II) entspricht, in der $R_1$ und $R_2$, identisch oder verschieden, ein Wasserstoffatom oder eine Elektronendonatorgruppe in 4,4′-Position und $n_1$ und $n_2$, identisch oder verschieden, gleich 0 oder 1 sind.

4. Verfahren nach einem der Ansprüche 1 bis 3, dadurch gekennzeichnet, daß die Keto-Verbindung der allgemeinen Formel (II) entspricht, in der $R_1$ und $R_2$, identisch oder verschieden, ein Wasserstoffatom, eine Methyl-, Ethyl-, tert.-Butyl- oder Phenylgruppe, einen Methoxy- oder Ethoxyrest oder eine Hydroxylgruppe darstellen, vorzugsweise in 3,3′- oder 4,4′-Position.

5. Verfahren nach einem der Ansprüche 1 bis 4, dadurch gekennzeichnet, daß die Keto-Verbindung der allgemeinen Formel (II)
   - Benzophenon
   - 2-Methylbenzophenon
   - 2,4-Dimethylbenzophenon
   - 4,4′-Dimethylbenzophenon
   - 2,2′-Dimethylbenzophenon
   - 4,4′-Dimethoxybenzophenon
   - Fluorenon
   - 4-Hydroxybenzophenon

- 4,4'-Dihydroxybenzophenon
- 4-Benzoylbiphenyl

ist.

6. Verfahren nach einem der Ansprüche 1 bis 5, dadurch gekennzeichnet, daß die Menge an Keto-Verbindung der Formel (II) mindestens $1.10^{-3}$ Mol pro Mol Wasserstoffperoxid beträgt, und vorzugsweise zwischen $1.10^{-3}$ und 10 Mol pro Mol Wasserstoffperoxid, insbesondere zwischen 0,05 und 1,0 Mol, liegt.

7. Verfahren nach einem der Ansprüche 1 bis 6, dadurch gekennzeichnet, daß die starke Säure einen pKa-Wert in Wasser von kleiner als -0,1, vorzugsweise kleiner als -1,0, hat.

8. Verfahren nach einem der Ansprüche 1 bis 7, dadurch gekennzeichnet, daß die starke Säure eine halogenierte oder nichthalogenierte Sauerstoffsäure ist, wie z.B. Schwefelsäure, Pyroschwefelsäure, Perchlorsäure, Salpetersäure oder eine Sulfonsäure wie beispielsweise Fluorsulfonsäu- re, Chlorsulfonsäure, Trifluormethansulfonsäure, Methansulfonsäure, Ethansulfonsäure, Ethandisulfonsäure, Benzolsulfonsäure, Benzoldisulfonsäuren, Toluolsulfonsäuren, Naphtalinsulfonsäuren oder Naphtalindisulfonsäuren.

9. Verfahren nach einem der Ansprüche 1 bis 8, dadurch gekennzeichnet, daß die starke Säure Perchlorsäure oder Trifluormethansulfonsäure ist.

10. Verfahren nach einem der Ansprüche 1 bis 9, dadurch gekennzeichnet, daß die Menge an starker Säure so dosiert ist, daß das Verhältnis von $H^+/H_2O_2$ zwischen $1.10^{-4}$ und 1,0, vorzugsweise zwischen $1.10^{-3}$ und 0,1, liegt.

11. Verfahren nach einem der Ansprüche 1 bis 6, dadurch gekennzeichnet, daß man in Gegenwart einer wirksamen Menge eines Alkali- oder Erdalkalisalzes einer starken Säure und einer wirksamen Menge mindestens einer Phosphorsauerstoffsäure verfährt.

12. Verfahren nach Anspruch 11, dadurch gekennzeichnet, daß das Alkali- oder Erdalkalisalz der starken Säure ein halogeniertes oder nichthalogeniertes Salz einer Sauerstoffsäure, wie z.B. Schwefelsäure, Pyroschwefelsäure, Perchlorsäure, Salpetersäure oder eine Sulfonsäure wie beispielsweise Fluorsulfonsäure, Chlorsulfonsäure, Trifluormethansulfonsäure, Methansulfonsäure, Ethansulfonsäure, Ethandisulfonsäure, Benzolsulfonsäure, Benzoldisulfonsäuren, Toluolsulfonsäuren, Naphtalinsulfonsäuren oder Naphtalindisulfonsäuren, ist.

13. Verfahren nach einem der Ansprüche 11 und 12, dadurch gekennzeichnet, daß das Alkalisalz Dinatriumsulfat, Natriumperchlorat, Natriumtrifluormethansulfonat, Natriumparatoluolsulfonat, Natriumchlorsulfonat, Natriumfluorsulfonat oder Natriummethansulfonat ist.

14. Verfahren nach einem der Ansprüche 11 bis 13, dadurch gekennzeichnet, daß das Alkalisalz Calciumsulfat, Magnesiumsulfat, Calciumperchlorat, Magnesiumperchlorat, Calciumtrifluormethansulfonat, Magnesiumtrifluormethansulfonat, Calciumparatoluolsulfonat, Magnesiumparatoluolsulfonat, Calciumfluorsulfonat, Magnesiumfluorsulfonat, Calciummethansulfonat oder Magnesiummethansulfonat ist.

15. Verfahren nach Anspruch 11, dadurch gekennzeichnet, daß die Phosphorsauerstoffsäuren saure Verbindungen von Phosphor in der Oxidationsstufe 5 sind.

16. Verfahren nach Anspruch 15, dadurch gekennzeichnet, daß die Phosphor-(V)-Sauerstoffsäuren Orthophosphorsäure, Methaphosphorsäure, Pyrophosphorsäure, Polyphosphorsäuren oder Phosphonsäuren wie beispielsweise 1-Hydroxyethylidendiphosphonsäure, Phosphonsäure, Ethylphosphonsäure oder Phenylphosphonsäure sind.

17. Verfahren nach einem der Ansprüche 15 oder 16, dadurch gekennzeichnet, daß die Phosphorsauerstoffsäuren Orthophosphorsäure, Pyrophosphorsäure und 1-Hydroxyethylidendiphosphonsäure sind.

18. Verfahren nach einem der Ansprüche 11 bis 17, dadurch gekennzeichnet, daß die Menge an Alkali- oder Erdalkalisalz, ausgedrückt als molares Verhältnis von Alkali- oder Erdalkalisalz / Wasserstoffperoxid, zwischen 0,001 und 0,10, vorzugsweise zwischen 0,005 und 0,05, liegt.

19. Verfahren nach einem der Ansprüche 11 bis 18, dadurch gekennzeichnet, daß die Menge an Phosphorsauerstoffsäure , ausgedrückt als molares Verhältnis von Phosphorsauerstoffsäure / Wasserstoffperoxid, zwischen 0,001 und 0,20, vorzugsweise zwischen 0,005 und 0,10, liegt.

**20.** Verfahren nach einem der Ansprüche 1 bis 19, dadurch gekennzeichnet, daß das molare Verhältnis von $H_2O_2$ / Phenolverbindung der Formel (I) zwischen 0,01 und 0,3, vorzugsweise zwischen 0,05 und 0,10, liegt.

**21.** Verfahren nach einem der Ansprüche 1 bis 11, dadurch gekennzeichnet, daß man in Gegenwart eines Komplexierungsmittels für Übergangsmetallionen verfährt, die unter den Reaktionsbedingungen - wie z.B. Phosphorsäuren, Polyphosphorsäuren, Phosphonsäuren oder deren Estersäuren - stabil sind.

**22.** Verfahren nach einem der Ansprüche 1 bis 21, dadurch gekennzeichnet, daß man bei einer Temperatur zwischen 45 und 150°C, vorzugsweise zwischen 45 und 75°C, verfährt.

**23.** Verfahren nach einem der Ansprüche 1 bis 22, dadurch gekennzeichnet, daß die Phenolverbindung der Formel (I) Phenol, Anisol, Orthokresol, Parakresol, Metakresol, tert.-4-Butylphenol, 2-Methoxyphenol oder 4-Methoxyphenol ist.

**24.** Verfahren nach einem der Ansprüche 1 bis 23, dadurch gekennzeichnet, daß die Phenolverbindung der Formel (I) Phenol ist.

## Claims

**1.** Process for the hydroxylation of phenolic compounds of general formula (I):

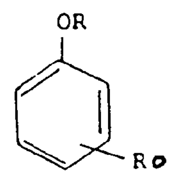

(I)

in which R and Ro, which may be identical or different, represent a hydrogen atom, an alkyl radical having from 1 to 4 carbon atoms, a cyclohexyl radical or a phenyl radical, by means of hydrogen peroxide in the presence of an effective amount of a strong acid, the said process being characterised in that the reaction is performed in the presence of a keto compound corresponding to the general formula (II):

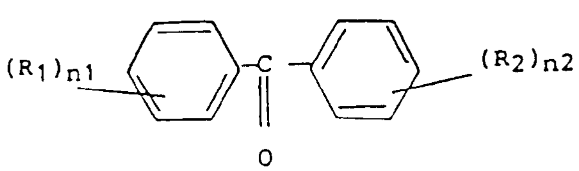

(II)

in the said formula (II):

- $R_1$ and $R_2$, which may be identical or different, represent a hydrogen atom or an electron-donating group,
- $n_1$ and $n_2$, which may be identical or different, are numbers equal to 0, 1, 2 or 3,
- optionally, the two carbon atoms located at the α-position with respect to the two carbon atoms carrying the -CO group may be linked together via a valency bond or via a $-CH_2-$ group, thereby forming a keto-containing ring which can be either saturated or unsaturated.

**2.** Process according to Claim 1, characterised in that the keto compound corresponds to the general formula (II) in which $R_1$ and $R_2$, which may be identical or different, represent:

- linear or branched alkyl radicals having from 1 to 4 carbon atoms,
- a phenyl radical,
- alkoxy radicals $R_3$-O- in which $R_3$ represents a linear or branched alkyl radical having from 1 to 4 carbon atoms or a phenyl radical,

- a hydroxyl group,
- a fluorine atom.

3. Process according to one of Claims 1 and 2, characterised in that the keto compound corresponds to the general formula (II) in which $R_1$ and $R_2$, which may be identical or different, represent a hydrogen atom or an electron-donating group at the 4,4′-position and $n_1$ and $n_2$, which may be identical or different, are equal to 0 or 1.

4. Process according to one of Claims 1 to 3, characterised in that the keto compound corresponds to the general formula (II) in which $R_1$ and $R_2$, which may be identical or different, represent a hydrogen atom; a methyl, ethyl, tert-butyl or phenyl radical; a methoxy or ethoxy radical; or a hydroxyl group, preferably at the 3,3′- or 4,4′-position.

5. Process according to one of Claims 1 to 4, characterised in that the keto compound of general formula (II) is:
   - benzophenone
   - 2-methylbenzophenone
   - 2,4-dimethylbenzophenone
   - 4,4′-dimethylbenzophenone
   - 2,2′-dimethylbenzophenone
   - 4,4′-dimethoxybenzophenone
   - fluorenone
   - 4-hydroxybenzophenone
   - 4,4′-dihydroxybenzophenone
   - 4-benzoylbiphenyl

6. Process according to one of Claims 1 to 5, characterised in that the amount of keto compound of formula (II) is at least 1 x $10^{-3}$ mole per mole of hydrogen peroxide, and preferably between 1 x $10^{-3}$ mole and 10 moles per mole of hydrogen peroxide, and still more preferably between 0.05 and 1.0 mole.

7. Process according to one of Claims 1 to 6, characterised in that the strong acid is an acid possessing a pKa in water of less than -0.1, and preferably less than -1.0.

8. Process according to one of Claims 1 to 7, characterised in that the strong acid is an oxoacid, halogenated or otherwise, such as sulphuric acid, pyrosulphuric acid, perchloric acid, nitric acid or a sulphonic acid such as fluorosulphonic acid, chlorosulphonic acid, trifluoromethanesulphonic acid, methanesulphonic acid, ethanesulphonic acid, ethanedisulphonic acid, benzenesulphonic acid, benzenedisulphonic acids, toluenesulphonic acids, naphthalenesulphonic acids and naphthalenedisulphonic acids.

9. Process according to one of Claims 1 to 8, characterised in that the strong acid is perchloric acid or trifluoromethanesulphonic acid.

10. Process according to one of Claims 1 to 9, characterised in that the amount of strong acid is such that the $H^+/H_2O_2$ ratio is between 1 x $10^{-4}$ and 1.0, and preferably between 1 x $10^{-3}$ and 0.1.

11. Process according to one of Claims 1 to 6, characterised in that the reaction is performed in the presence of an effective amount of an alkali metal salt or alkaline earth metal salt of a strong acid and an effective amount of at least one phosphorus oxoacid.

12. Process according to Claim 11, characterised in that the alkali metal salt or alkaline earth metal salt of a strong acid is a salt of an oxoacid, halogenated or otherwise, such as sulphuric acid, pyrosulphuric acid, perchloric acid, nitric acid or a sulphonic acid such as fluorosulphonic acid, chlorosulphonic acid, trifluoromethanesulphonic acid, methanesulphonic acid, ethanesulphonic acid, ethanedisulphonic acid, benzenesulphonic acid, benzenedisulphonic acids, toluenesulphonic acids, naphthalenesulphonic acids and naphthalenedisulphonic acids.

13. Process according to one of Claims 11 and 12, characterised in that the alkali metal salt is disodium sulphate, sodium perchlorate, sodium trifluoromethanesulphonate, sodium para-toluenesulphonate, sodium chlorosulphonate, sodium fluorosulphonate or sodium methanesulphonate.

14. Process according to one of Claims 11 to 13, characterised in that the alkaline earth metal salt is calcium sulphate, magnesium sulphate, calcium perchlorate, magnesium perchlorate, calcium trifluoromethanesulphonate, magnesium trifluoromethanesulphonate, calcium para-toluenesulphonate, magnesium para-toluenesulphonate, calcium fluorosulphonate, magnesium fluorosulphonate, calcium methanesulphonate or magnesium methanesulphonate.

15. Process according to Claim 11, characterised in that the phosphorus oxoacids are compounds having an acid function of phosphorus with the oxidation number 5.

16. Process according to Claim 15, characterised in that the phosphorus V oxoacids are orthophosphoric acid, metaphosphoric acid, pyrophosphoric acid, polyphosphoric acids and phosphonic acids such as (1-hydroxyethylidene)-diphosphonic acid, phosphonic acid, ethylphosphonic acid and phenylphosphonic acid.

17. Process according to one of Claims 15 and 16, characterised in that the phosphorus oxoacids are orthophosphoric acid, pyrophosphoric acid and (1-hydroxyethylidene)diphosphonic acid.

18. Process according to one of Claims 11 to 17, characterised in that the amount of alkali metal salt or alkaline earth metal salt, expressed as the mole ratio of alkali metal salt or alkaline earth metal salt to hydrogen peroxide, is between 0.001 and 0.10, and preferably between 0.005 and 0.05.

19. Process according to one of Claims 11 to 18, characterised in that the amount of phosphorus oxoacid, expressed as the phosphorus oxoacid/hydrogen peroxide mole ratio, is between 0.001 and 0.20, and preferably between 0.005 and 0.10.

20. Process according to one of Claims 1 to 19, characterised in that the mole ratio of $H_2O_2$ to phenolic compound of formula (I) is between 0.01 and 0.3, and preferably between 0.05 and 0.10.

21. Process according to one of Claims 1 to 11, characterised in that the reaction is performed in the presence of an agent which complexes transition metal ions and which is stable under the reaction conditions, such as phosphoric acids, polyphosphoric acids, phosphonic acids and their acid esters.

22. Process according to one of Claims 1 to 21, characterised in that the reaction is performed at a temperature of between 45° and 150°C and preferably between 45° and 75°C.

23. Process according to one of Claims 1 to 22, characterised in that the phenolic compound of formula (I) is phenol, anisole, ortho-cresol, para-cresol, meta-cresol, 4-tert-butylphenol, 2-methoxyphenol or 4-methoxyphenol.

24. Process according to one of Claims 1 to 23, characterised in that the phenolic compound of formula (I) is phenol.